(19) **Europäisches Patentamt / European Patent Office / Office européen des brevets**

(11) **EP 4 234 029 A1**

(12) **EUROPEAN PATENT APPLICATION**
published in accordance with Art. 153(4) EPC

(43) Date of publication:
**30.08.2023 Bulletin 2023/35**

(21) Application number: **21806135.6**

(22) Date of filing: **28.09.2021**

(51) International Patent Classification (IPC):
**A61P 35/00** (2006.01)   **C07K 14/705** (2006.01)
**C07K 16/28** (2006.01)

(52) Cooperative Patent Classification (CPC):
**A61P 35/00; A61K 39/001111; C07K 14/70503;
C07K 14/70532; C07K 16/2827;** A61K 2039/505;
A61K 2039/54; A61K 2039/545

(86) International application number:
**PCT/CU2021/050008**

(87) International publication number:
**WO 2022/083805 (28.04.2022 Gazette 2022/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.10.2020 CU 20200075**

(71) Applicant: **Centro de Ingeniería Genética y
Biotecnología
La Habana 11300 (CU)**

(72) Inventors:
  • **MORERA DÍAZ, Yanelys
    Playa La Habana 11300 (CU)**
  • **SÁNCHEZ RAMÍREZ, Javier
    Plaza de la Revolución La Habana 10400 (CU)**
  • **CANAÁN-HADEN AYALA, Camila
    Playa La Habana 11300 (CU)**

  • **BEQUET ROMERO, Mónica
    Plaza de la Revolución La Habana 10400 (CU)**
  • **AYALA ÁVILA, Marta
    Playa La Habana 11300 (CU)**
  • **GONZALEZ MOYA, Isabel
    Playa La Habana 11300 (CU)**
  • **GONZALEZ BLANCO, Sonia
    Playa La Habana 11300 (CU)**
  • **LIMONTA FERNÁNDEZ, Miladys
    Playa La Habana 11300 (CU)**
  • **ESPINOSA RODRÍGUEZ, Luis, Ariel
    Marianao La Habana 11500 (CU)**
  • **BESADA PÉREZ, Vladimir, Armando
    Playa La Habana 11300 (CU)**

(74) Representative: **Capitán García, Maria Nuria
Felipe IV no. 10, bajo iz.
28014 Madrid (ES)**

Remarks:
The complete document including Reference
Table(s) and the Sequence Listing(s) can be
downloaded from the EPO website

(54) **CHIMERIC ANTIGEN COMPRISING THE EXTRACELLULAR DOMAIN OF PD-L1**

(57) Chimeric antigen comprising multimeric aggregates of the extracellular domain of the programmed death ligand 1 (PD-L1) with a reduced binding capacity to the PD-1 and CD80 receptors as compared to the native PD-L1 molecule. The invention further discloses pharmaceutical compositions including said chimeric antigen and at least a pharmaceutically acceptable vaccine adjuvant. The chimeric antigen is used for the manufacturing of a drug to treat cancer or its metastases. The invention also discloses a method of treating cancer or its metastases in a subject in need thereof, characterized by the administration of a therapeutically effective amount of the pharmaceutical composition comprising the chimeric antigen described herein.

EP 4 234 029 A1

**Description**

**Technical field**

The invention is related to the fields of biotechnology and human health. It provides the description of a chimeric vaccine antigen comprising more than 200 amino acids of the extracellular domain of the PD-L1 protein. The invention also provides a vaccine composition comprising said antigen and at least one adjuvant. This composition induces a PD-L1-specific immune response, with neutralizing activity on the PD-L1-mediated biological effects; and therefore could be used for the treatment of cancer and its metastases.

**Background of the invention**

Programmed death-ligand 1, known as PD-L1, is a 40 kDa type 1 transmembrane protein receptor, member of the B7 family. PD-L1 has specific and functional interaction with the programmed cell death protein 1 (PD-1) and also with another membrane receptor, known as CD80. PD-L1 is a 290-amino acid protein encoded by the Cd274 gene, located on chromosome 9 of the human genome. In normal tissues, PD-L1 is expressed on T cells, B cells, myeloid cells, dendritic cells, non-hematopoietic cells from non-lymphoid organs, but also is expressed in a wide types of tumors (Wang, et al., OncoTargets and Therapy, 2016: 9: 5023-39, Salmaninejad, et al., Journal of Cellular Physiology, 2019: 234: 16824-37).

The PD-1/PD-L1 interaction is involved in different mechanisms related to the tumor-induced immunosuppressive environment avoiding the immune system attack. The binding between PD-1 on T cells and PD-L1 on tumor cells induces the suppression of the immune response by decreasing the proliferation and differentiation of T lymphocytes. In addition, this interaction induces the activation of T-cell apoptosis, anergy and their functional exhaustion. PD-L1 is overexpressed in many malignant tumors and is associated with a poor prognosis.

The use of immunotherapeutic agents such as anti-PD-L1 monoclonal antibodies that block the interaction of the molecule with this natural receptors has been considered a promising alternative in cancer treatment. Within this group of antibodies, the FDA has approved atezolizumab, durvalumab and avelumab to treat patients with several cancer types, particularly in advanced-stage tumors, including metastatic urothelial carcinoma, Merkel cell carcinoma, squamous non-small cell lung cancer and renal cell carcinoma (Lee, et al., Molecules, 2019: 24: 1190). Treatment with anti-PD-L1 monoclonal antibodies shows some clinical success, but requires frequent administration and high doses of the therapeutic, which leads to high costs and adverse effects such as pneumonitis, myocarditis, colitis, among others (Wang y Xu, JAMIA Open, 2018: 2: 173-8). In many cases, the severity of these toxicities causes the delay or discontinuation of passive therapy.

On the other hand, others strategies tried to address these issues by taking active immunotherapy approaches based on PD-L1 as an antigen, such as those that used polypeptides covering most of the protein or their peptides. Munir *et al* performed *in vitro* tests with two PD-L1 peptides, and evaluated their ability to stimulate peripheral blood mononuclear cells isolated from advanced melanoma cancer patients, treated with a vaccine called DCvacc. The vaccine involved the administration to the patients of dendritic cells transfected with mRNA encoding the tumor-associated antigens p53 and telomerase. Peripheral blood mononuclear cells from these patients had only limited reactivity toward DCvacc. However, when these cells were stimulated *in vitro* with two PD-L1-derived peptides called as PDLong1 (identified herein as SEQ ID NO: 23) and PDLong2 (identified herein as SEQ ID NO: 24), the T cell reactivity toward the vaccine was significantly increased (Munir, et al., Oncoimmunology, 2013: 2: e23991).

Based on the aforementioned finding, and other additional data, a peptide vaccine directed to PD-L1 was developed using the PDLong1 peptide as antigen. This peptide, which contains epitopes for CD4+ and CD8+ T lymphocytes, was mixed with the adjuvant Montanide ™, and administered to patients with multiple myeloma in a phase I clinical study. This antigen preparation was safe and immunogenic in humans. Lymphocytes obtained from the skin biopsies of the vaccinated cancer patients were activated in the presence of the peptide. However, PD-L1-specific antibody response was not detected in immunized patients (Jørgensen, et al., HemaSphere, 2019: 3: 631-2). This element could represent a limitation, since it has been demonstrated that the antibody response is a relevant factor in the efficacy of this type of treatment. Antibodies against this ligand are able to block the interactions between PD-L1 and its receptors, and also enhance T-cell mediated anticancer immunity.

An active immunotherapy strategy based on a deoxyribonucleic acid (DNA) vaccine has also been developed. This DNA vaccine is administered by the oral route using a live attenuated bacterium (*Salmonella typhimurium* species) transformed with a plasmid encoding the extracellular domain of PD-L1. This strategy was disclosed in patent application WO 2018/167290. Taking advantage of the bacterial infection mechanism, cytotoxic CD8+ lymphocytes were generated and activated by dendritic cells that presented PD-L1 peptides associated with the major histocompatibility complex class I. These cytotoxic lymphocytes were able to eliminate PD-L1 positive tumor cells. In addition to the activation of the cellular branch of the immune response, this vaccine also induced an antibody response specific to PD-L1. Nevertheless, the elicited antibody levels were relatively low (Wieckowski, et al., Journal of Clinical Oncology, 2018: 36: 74). This element could be unfavorable for the success of this variant of active immunotherapy.

Active immunotherapy with a fusion polypeptide based on the extracellular domain of PD-L1 (Phe 19-Glu 228) and diphtheria toxin (DTT) was described in patent application WO 2014/183649. This second polypeptidic segment was included to promote a strong CD4+ T cell-mediated immune response. The extracellular domain of PD-L1 was produced in the GST (Glutathione S-transferase) expression system. This expression system guarantees its correct folding and three-dimensional conformation, which ensures the structural integrity of the fusion protein. The vaccine antigen (43.5 kDa) mixed with Freund's adjuvant induced PD-L1-specific antibody response and cytotoxic T lymphocytes. The effectors of this immune response inhibited tumor growth and metastasis implantation (Lin, et al., Molecular Therapy Oncolytics, 2019: 14: 222-32). However, the levels of elicited antibodies could be considered as relatively moderate, taken into account the use of Freund's adjuvant, currently considered the strongest adjuvant.

Therefore, the development of new PD-L1-based vaccine antigens with a higher immunogenicity as well as abrogated pro-tumoral activity, remains as an area of interest. This strategy must be able to generate simultaneously a vigorous humoral and cellular immune response, without the need to increase the amounts of antigen to be administered.

**Disclosure of the invention**

This invention solves the previously mentioned problem by providing a chimeric antigen that comprises the extracellular domain of the programmed death ligand 1 (PD-L1) which forms multimeric aggregates with a reduced binding capacity to the PD-1 and CD80 receptors as compared to the native PD-L1 protein. Said chimeric antigen is structurally aggregated, which makes that the conformation of the extracellular domain of PD-L1 present in this polypeptide is modified, in comparison with the conformation of said domain in the native ligand.

In the present invention, the extracellular domain of human PD-L1 refers to the region between amino acids Phe 19 and Arg 238 of PD-L1. In one embodiment of the invention, the chimeric antigen comprising the human extracellular domain of PD-L1 also comprises an amino terminal to increase its expression in bacteria and a carboxyl terminal to facilitate its purification by affinity chromatography. In one embodiment of the invention, the chimeric antigen has an amino acid sequence identified as SEQ ID NO: 1 or an amino acid sequence having at least 95% of identity with SEQ ID NO: 1.

The antigen identified as SEQ ID NO: 1 in the Sequence Listing includes the first 47 amino acids of the amino terminal of LpdA protein from *Neisseria meningitidis,* the extracellular domain of the PD-L1 molecule (from Phe 19 to Arg 238). Both regions are separated by 13 amino acids that function as a linker between both polypeptides. The protein of interest of this invention was obtained starting from the assembly of the gene by the polymerase chain reaction technique, using 18 partially overlapping oligonucleotides. For cloning in the expression vector, primers designed according to the characteristics of the expression vector were employed, where the DNA was inserted and finally sequenced. The sequence was compared with the one reported for the extracellular domain of PD-L1, published in the Uni-ProtKB/Swiss-Prot databases with the identifier Q9NZQ7 (https://www.uniprot.org/uniprot/Q9NZQ7) and its identity was confirmed. The polypeptide obtained by the method described above can be cloned into expression vectors in viruses, bacteria, yeasts, phages, plants or mammalian cells, changing the insertion sites accordingly. Once the genetic construction has been obtained, its sequence must be verified using traditional automatic sequencing methods.

In a particular embodiment, to obtain the chimeric polypeptide, the pM238 *Escherichia coli* expression vector was used, which contains, among other elements, the tryptophan promoter, a nucleotide sequence coding for 47 amino acids corresponding to the amino terminal of the LpdA protein of *N. meningitidis* (Patent EP0816506B1), a nucleotide sequence coding for a segment of six histidines and the T4 terminator. This expression vector also carries the ampicillin resistance gene, as a selection marker. The fusion polypeptide obtained was named PKPD-L1.

Once the chimeric polypeptide was purified, an analysis by size exclusion HPLC was performed, which showed a major peak eluting in the void volume of the column, accounting for 90% of the total protein applied to the column. This major peak contains aggregated protein PKPD-L1 with a molecular weight greater than 670 kDa, which differs from the molecular weight of 26 kDa corresponding to the extracellular domain of the PD-L1 native protein (Example 2). Surprisingly, this fusion polypeptide has changes in its structure that led to the formation of high molecular weight aggregates. The stable formation of these aggregates results in a PD-L1 variant with the following characteristics: (1) an spatial arrangement of several PD-L1 molecules that modifies the particular stoichiometry of the natural PD-1/PD-L1 or CD80/PD-L1 interactions, causing that the fusion polypeptides have a reduced binding to said receptors, in comparison with a variant of native PD-L1 biologically active; (2) the binding of several PD-L1 molecules leads to the repeated exposure of epitopes, as a consequence of said aggregation, which in turn causes an increase in immunogenicity, in comparison with the one obtained when PD-L1 is found as a monomer in its native three-dimensional structure. These findings support the development of a novel therapy, much more effective and safer, which can be applied in the control of cancer.

The invention discloses a pharmaceutical composition comprising the chimeric antigen comprising the extracellular domain of human PD-L1 (which forms a multimer with a reduced binding capacity to the PD-1 and CD80 receptors respect to the native form of PD-L1) and at least one pharmaceutically accepted vaccine adjuvant. The distinctive feature of the PKPD-L1 polypeptide of being structurally aggregated, and its enhanced immunogenicity when it is combined with an adjuvant, without compromising the safety profile, gives to this polypeptide immunological advantages, when compared with other PD-L1 variants. Its safety also presents advantages; as it is part of a vaccine that is repeatedly administered (due to the chronicity of the treatment) in comparison with the passive immunotherapy strategy with specific antibodies. These elements make PKPD-L1 an antigen with a high potential for the treatment of diseases associated to an increase PD-L1 expression. The present invention describes a specific active immunotherapy characterized by the administration of a structurally aggregated recombinant chimeric polypeptide representative of human PD-L1, which is combined with an adjuvant.

In one embodiment of the invention, in the pharmaceutical composition, the vaccine adjuvant is selected from the group composed by oil adjuvants, mineral salts, proteoliposomes and proteoliposomes conjugated to gangliosides. In order to stimulate the development of the immune response, the chimeric antigen of the present invention can be combined with immunopotentiators and/or adjuvants. The nature of these compounds is usually diverse, for example compounds of mineral salts (for example, aluminum hydroxide, aluminum phosphate, calcium phosphate); soluble cytokines (ie, IL-2, IL-15, IL-12, GM-CSF, IFN-gamma, IL-18); membrane receptors (ie CD40, CD154, the invariant chain of Major Histocompatibility Complex type I, LFA3); saponins (ie. QS21); oligonucleotides such as CpG; glycolipids such as lipopolysaccharides; emulsions (ie. Freund's adjuvant, synthetic adjuvant formulation (SAF), MF59, Montanide ™), liposomes, nanoparticles and virosomes; microparticulate adjuvants, poloxamers, adjuvants from viral origin (ie. HBcAg, HCcAg, HBsAg) and bacterial origin (ie. NAcGM3-VSSP, N-GliGM3-VSSP); mucosal adjuvants such as heat labile enterotoxin, cholera toxin, and mutant toxins (ie. LTK63 and LTR72).

The immunogen can be administered in vehicles accepted for pharmaceutical use that are not toxic nor exhibit adverse therapeutic effects, and that are known to those skilled in the art. The examples presented in this invention do not restrict in any way the use of a particular buffer or combinations of buffers, and even the use of excipients that contribute to increase its stability.

In another embodiment, the invention provides the use of the chimeric antigen that comprises the extracellular domain of human PD-L1 which forms a multimer that has a reduced binding capacity to the PD-1 and CD80 receptors, when compared to the native form of PD-L1, for the manufacture of a drug for the treatment of cancer or its metastases. It is due to the considerably stable conformation and the high molecular weight of the fusion polypeptide PKPD-L1, which increase the immunogenic properties, when compared to a non-fusion variant of the protein or a mixture of PD-L1 peptides. The fusion protein PKPD-L1, administered with the adjuvants aluminum phosphate or VSSP (*Very small size proteoliposomes,* patent US6149921; International Application WO201986056A1) originates a vaccine able to break immune tolerance against the autologous molecule, and able to induce a specific humoral and cellular immune response to native PD-L1. This immune response is superior, qualitatively and quantitatively, when compared to passive immunotherapy with monoclonal antibodies, or when is compared to the immune response generated by antigens based in the native PD-L1 protein or in a mixture of PD-L1 peptides. This increase in the PKPD-L1 polypeptide immunogenicity prompted an increment in the anti-tumor activity and it is superior to those generated by other evaluated strategies.

The inhibitory effects on tumor growth demonstrated in the invention may be based on different mechanisms, without ruling out their possible combinations. The polyclonal anti-PD-L1 antibody response generated by vaccination is capable of blocking the interaction between the PD-L1 ligand, expressed on the tumor cell surface, and PD-1, expressed in the membrane of cytotoxic CD8 + T lymphocytes, avoiding functional inactivation thereof. On the other hand, the antibodies generated by vaccination can decrease, through the internalization process, the levels of PD-L1 on the surface of the tumor cell. The decrease in PD-L1 could lead to a reduction in the malignant cell's ability to inactivate cytotoxic CD8 + T lymphocytes. Additionally, the low expression of the PD-L1 ligand in the tumor cell encourages it to acquire a phenotype similar to the epithelial one, and therefore, negatively modulates its metastatic capacity. The polyclonal nature of the antibody response is an important factor in the effectiveness of the biological events described above.

Furthermore, the immunization with PKPD-L1 induced specific cytotoxic lymphocytes against the primary tumor cells and their metastases, which potentially present PD-L1 peptides in the context of the Major Histocompability Complex class I. It is an advantage of the immunization with the PKPD-L1 fusion protein, that in addition to their capacity to generate a greater number of lymphocytes, it could activates both CD4 + and CD8 + T lymphocytes and thereby achieving a diversity of their clones. By immunizing with a chimeric protein that comprises more than 200 amino acids from the extracellular region of the PD-L1 protein, it is ensured: (1) the simultaneous presentation of linear epitopes being able to activate both: CD4+ helper lymphocytes and of cytotoxic CD8 + lymphocytes, which guarantee a more effective immune response against tumors; (2) the presence of several restricted epitopes for CD4+ helper lymphocytes and for cytotoxic CD8+ lymphocytes, which guarantee the presence of different cell clones for both cell types, which could also translate into a more effective immune response against tumors; (3) the presence of several epitopes also allow that, in the context of vaccination in a population of patients with different alleles of MHC class I or II, an increase in the number of individuals with specific anti-PD-L1 humoral and/or cellular response. These elements constitute beneficial advantages that do not exist or are very limited when the immunization involves peptides restricted to only one allele of the major histocompatibility complex. All these properties of the immune response that are generated after immunization with the PKPD-L1 protein acquire greater relevance when compared with the active immunotherapy strategy with the native PD-L1 molecule. An important element is that a superior immune response is achieved with the same amount of antigen. This feature implies that the immunotherapy strategy in humans involving PKPD-L1 has advantages from the productive and safety point of view. The PKPD-L1 polypeptide is a molecule with less biological activity, as compared to the native variant, and therefore the administration of high doses of antigen does not compromise its safety profile.

The invention also provides a method for the treatment of cancer or its metastases in an individual in need, characterized by the administration of a therapeutically effective amount of a pharmaceutical composition comprising a) the chimeric antigen comprising the extracellular domain of human PD-L1 which forms a multimer that has a reduced binding capacity to the PD-1 and CD80 receptors, when compared to the native form of PD-L1, and b) at least one pharmaceutically acceptable vaccine adjuvant. This method, which involves immunization with the aforementioned chimeric antigen, is able to reduce more effectively the development of the primary tumor and prevents the occurrence of distant metastases, which offers advantages for increasing the survival of cancer patients.

In one embodiment of the invention, the method involving the administration of the composition comprising the chimeric antigen is combined with passive immunotherapy or with standard cancer therapy applied simultaneously or sequentially. This treatment method can be used as first or second line therapy, in association or not with other chemical, radiological or biological anti-tumor agents.

In one embodiment of the invention, the administration of the vaccine comprising the chimeric antigen is combined with the bolus injection, or with the controlled release of monoclonal antibodies or their fragments specific for antigens relevant to tumor growth or for antigens involved in the tumor induced immunosuppression. The vaccine preparations comprising the chimeric antigen are administered to a mammal, preferably to a human, but without excluding their veterinary use, in a therapeutically effective amount. The antigen-adjuvant mixture, also known as an immunogen, can be administered by a variety of routes, including subcutaneous, intramuscular, mucosal, intraperitoneal, intralymphatic, topical, or by inhalation. In a preferred embodiment, the route of administration of the immunogen comprising the chimeric PKPD-L1 antigen is subcutaneous. The examples presented in this invention do not restrict the use of the antigen with an adjuvant or the use a particular route of administration.

The antigen doses to be used can be established according to different parameters. These doses depend on the route of administration, the pathology in question, and the treatment period. A change in the interval of administration of the doses, or a route of administration, different from those described in the examples that follow, does not depart from the essence of the present invention, being possible to achieve an optimization of the immunization schemes in order to obtain a better specific immune response to PD-L1.

In one embodiment of the invention, active immunotherapy with the chimeric antigen is combined with passive immunotherapy carried out with antibodies against the human PD-L1 ligand or against the human PD-1 receptor.

**Brief description of the figures**

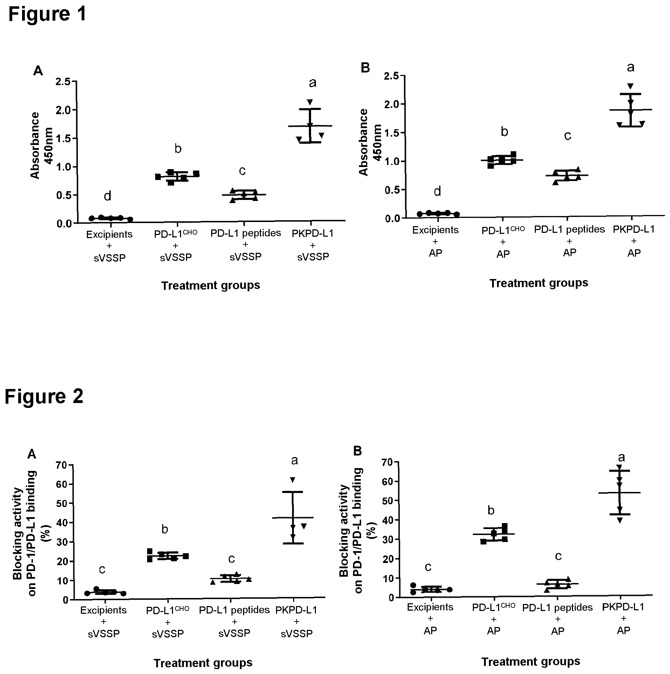

**Figure 1.** Antibody response generated in rats after immunization with the PKPD-L1 polypeptide in combination with the adjuvants sNAcGM3-VSSP (A) or aluminum phosphate (B), measured by ELISA. Antibody levels were expressed as absorbance units at 450 nm for the 1:250 sera dilutions. Different letters represent statistical significance according to the Tukey test.

**Figure 2.** Capacity of the sera from rats immunized with the PKPD-L1 protein in combination with the adjuvants sNAcGM3-VSSP (A) or aluminum phosphate (B) to block the interaction between the PD-1 receptor and its PD-L1 ligand; evaluated in a competitive ELISA.

**EXAMPLES**

**Example 1. Cloning and expression of the recombinant protein PKPD-L1 and the variant PD-L1$^{CHO}$.**

The DNA corresponding to the mature region of the extracellular domain of human PD-L1 was amplified, according to the sequence of the UniProtKB/Swiss-Prot database with identifier Q9NZQ7 (https://www.uniprot.org/uniprot/Q9NZQ7). Eighteen partially overlapping oligonucleotides were designed (from SEQ ID NO: 2 to SEQ ID NO: 19) The gene was amplified and assembled, using the polymerase chain reaction technique, using the KOD Hot Start Master Mix ™ reagent set (Novagen). After the assembly of the gene that codes for the extracellular domain of PD-L1, the restriction sites of the enzymes were added with the use of two primers designed to achieve this purpose. These primers contained the restriction sites for the enzymes Nhel and BamH I, respectively. Then, the resulting DNA was cloned into the pM238 expression vector, (SEQ ID NO: 20 and SEQ ID NO: 21). With the inclusion of the DNA sequence coding for mature region of PD-L1 (amino acids from Phe 19 to Arg 238) into the vector pM238, this segment was fused to DNA sequence coding for 47 amino acids from the amino terminal of the LpdA protein of *N. meningitidis* (Patent EP0816506). These regions are connected by a DNA coding for a linker of 13 amino acids. Towards the carboxyl terminal end of the fusion polypeptide, a region of six histidines was added, to facilitate its purification. The plasmid resulting from cloning was named pPKPD-L1. The nucleotide sequence of this plasmid was 100% verified. In the Sequence List, the nucleotide sequence of the coding region for the recombinant protein PKPD-L1 is identified as SEQ ID NO: 22. The fusion polypeptide was called PKPD-L1, and its amino acid sequence is identified in the Sequence List as SEQ ID NO: 1.

The recombinant plasmid PKPD-L1 was transformed into the E. coli BL21 strain. The bacteria were cultured at 37 °C in 5L of M9 media supplemented with 10% casein hydrolysate, glucose 40 % and 100 μg/mL ampicillin in a 5-L bioreactor for 3 hours. The pH was maintained to 7.0. To induce of the expression of the PKPD-L1, 3b-Indole-acrylic acid was added to a final concentration of 40 ug/ml and the culture was allowed to grow at 28 °C for 16 hours.

In order to obtain a polypeptide, representative of native PD-L1, a genetic construction was made for its expression in mammalian cells. This native PD-L1 variant (called PD-L1[CHO]) was obtained by transfecting CHO cells with DNA encoding the extracellular domain of the ligand (Phe 19 to Glu 238) inserted into the pcDNA 3.1/myc-His C vector (Invitrogen). The cell culture supernatant was harvested seven days after reaching 100% confluence. The protein was purified by immobilized metal affinity chromatography on a Ni-NTA resin (Qiagen).

## Example 2. Obtention, purification and evaluation of the state of aggregation of the PKPD-L1 polypeptide.

The bacterial pellet (described in Example 1) was sonicated to disrupt the cellular structure in phosphate buffer (50 mM $NaH_2PO_4$; 300 mM NaCl; pH 7.8). The insoluble fraction after cell disruption was solubilized in the phosphate buffer, under denaturing conditions with 6 M urea. The soluble fraction was used for PKPD-L1 protein purification by metal chelate affinity chromatography in a Ni-NTA matrix followed by gel filtration.

To demonstrate the integrity of the N- and C-terminal ends and verify the amino acid sequence, the protein was characterized by electrospray ionization mass spectrometry in an orthogonal hybrid QTOF-2TM tandem mass spectrometer. The ESI-MS and MS/MS spectra confirmed that amino acid sequence of the protein showed coincidence with the deduced from the cDNA. Ninety-four percent of the amino acid sequence was verified. Glu-C peptides containing the N- and C-terminal ends of the protein were sequenced and their sequenced agreed with the expected sequence.

With the purpose of estimate the PKPD-L1 molecular weight was used a size exclusion analytic HPLC in a Superdex™ 200 (GE Healthcare Life Sciences™) column, previously equilibrated with PBS. Molecular weight was estimated using the retention times, in comparison with a gel filtration standard (BioRad™) (Table 1).

**Table 1.** Molecular weight estimation of the PD-L1[CHO] and PKPD-L1 polypeptides using size-exclusion chromatography.

| Protein | Molecular weight (kDa) | Retention time (minutes) |
| --- | --- | --- |
| Bovine thyroglobulin[a] | 670.0 | 15.9 |
| Bovine γ globulin[a] | 158.0 | 23.1 |
| Chicken ovalbumin[a] | 44.0 | 26.3 |
| Equine myoglobin[a] | 17.0 | 29.3 |
| Vitamin B12[a] | 1.35 | 36.4 |
| PD-L1[CHO] | 32.0[b] | 27.0 |
| PKPD-L1 | 1078.0[b] | 15.7 |

[a]: Gel filtration standard components of known molecular weight. [b]: molecular weight estimated from the standard curve.

A major peak eluting in the void volume of the column and accounting for 90% of the applied protein PKPD-L1 was obtained. This peak contains protein aggregates with molecular weights higher than 670 kDa (highest molecular weight from the standard). Based on the interpolation results from the curve generated using the retention times and the known molecular weights of the standard components, the estimated molecular weight of PKPD-L1 aggregate is around 1078 kDa. This value differs from the theoretical molecular weight for the monomer of the PKPD-L1 polypeptide (31 kDa). Therefore, it could be inferred that the preparation is composed by multimers of the fusion polypeptide. The estimated molecular weight for the PKPD-L1 aggregate (1078 kDa) is also higher than the calculated molecular weight for the ligand PD-L1[CHO] (32 kDa), which is obtained in their native conformation and it is a biologically active molecule.

**Example 3. Evaluation of the binding capacity of the PKPD-L1 protein to the PD-1 and CD80 receptors.**

The ability of the chimeric protein PKPD-L1 to bind to the PD-1 and CD80 receptors was measured by an indirect ELISA using biologically active variants of these molecules as Fc-fusion proteins. PD-1/Fc (RγD Systems™) or CD80/Fc (RγD Systems™) proteins were immobilized on ELISA plates. Subsequently, the PD-L1/Fc (RγD Systems™), PKPD-L1 and PD-L1$^{CHO}$ proteins were added at different concentrations. Finally, to detect the binding of the receptors to their ligand, a human PD-L1 biotinylated antibody (RγD Systems™) was added followed by a streptavidin-peroxidase conjugate (Sigma™).

Table 2 shows that the binding of PD-1/Fc and CD80/Fc to the PD-L1/Fc and PD-L1$^{CHO}$ were similar, indicated that the PD-L1$^{CHO}$ protein, expressed in mammal's cells, has similar binding properties as compare with the commercially available biologically active protein. Nevertheless, the binding of PKPD-L1 polypeptide to the PD-1 receptor was 1420 times weaker than the interaction between the PD-L1/Fc molecules with PD-1. Additionally, the PKPD-L1 binding to the CD80 receptor was 689 times lower than the same binding of the PD-L1/Fc to the same molecule.

**Table 2.** Half maximal effective concentration ($EC_{50}$) for the interaction of PD-1/Fc and CD80/Fc with the PD-L1 variant molecules.

|  | PD-1/Fc | CD80/Fc |
|---|---|---|
| **PD-L1/Fc** | 201 ng/mL | 529 ng/mL |
| **PD-L1$^{CHO}$** | 231 ng/mL | 540 ng/mL |
| **PKPD-L1** | 284901 ng/mL | 364452 ng/mL |

These results indicate that the PKPD-L1 polypeptide has a reduced capacity to interact with the PD-1 and CD80 receptors, as compared to the biologically active native PD-L1 variant. Possibly, the spatial arrangement of many PKPD-L1 monomers, which form the high molecular weight aggregate, alters the particular stoichiometry of the PD-1/PD-L1 or CD80/PD-L1 bindings. This property is favorable, because it could reduce the risk associated with administering a protein with high pro-tumoral activity.

**Example 4. Antibody response generated after immunization with the PKPD-L1 protein.**

To evaluate the immunogenicity of the PKPD-L1 polypeptide, female Wistar rats were immunized subcutaneously with this polypeptide in combination with two adjuvants. A mixture of two PD-L1 synthetic peptides (identified as SEQ ID NO: 23 and SEQ ID NO: 24 in the Sequence List) conjugated to the LpdA protein of *N. meningitides* were also evaluated. The biologically active polypeptide PD-L1$^{CHO}$ was additionally included in this experiment. In all cases, each rat received 200 μg of polypeptide or conjugated per dose. Aluminum phosphate (Brenntag Biosector™) and sNAcGM3-VSSP (an adjuvant developed by the Center for Molecular Immunology, Havana, Cuba) were used as adjuvants. As negative control, a group of rats were immunized with the excipient (40 mM Tris pH 8.0) in combination with each adjuvant.

The immunization schedule with sNAcGM3-VSSP adjuvant (200 μg/dose) comprised eight weekly vaccinations, meanwhile using aluminum phosphate (0.7 mg of $AL^{3+}$/dose), the schedule included four bi-weekly administrations. Sera were collected one week after the last immunization (depending on each adjuvant).

An indirect ELISA was developed to detect specific antibodies to human PD-L1. The PD-L1/ Fc protein (RγD Systems ™) was captured with a polyclonal sheep antibody specific to the Fc region of a human IgG, immobilized on an ELISA plate. The rat sera were added at 1:250 dilution. Finally, to detect the PD-L1 specific antibodies a rat IgG-specific polyclonal antibody (Sigma™) was used.

Figure 1 shows the results from the evaluation of the levels of IgG antibodies specific to human PD-L1, expressed in absorbance units. The administration of the PKPD-L1 polypeptide (which forms high molecular weight aggregates), in combination with the aforementioned adjuvants, was able to generate a specific antibody against the PD-L1/Fc biologically active protein. The antibodies levels generated by immunization with the PKPD-L1 polypeptide in combination with sNAcGM3-VSSP were significantly higher than those induced by the administration of PD-L1$^{CHO}$ (Tukey's test, p <0.0001) and for the PD-L1 peptide mixture (Tukey's test, p < 0.0001). Similar results were obtained when the aluminum phosphate adjuvant was used; the specific antibody titers generated by the polypeptide PKPD-L1 were higher than those induced by the other two studied variants (Tukey's test, p <0.0001). These results indicate that the PKPD-L1 polypeptide has a higher immunogenicity as compare with a mixture of PD-L1 synthetic peptides or the PD-L1 native molecule. The increase of the PKPD-L1 antigenic properties can be explained by the high aggregation of the fusion polypeptide. With this aggregation, there is an increase in the number of antigenic determinants, with respect to the number that can be found within the native PD-L1 protein. In addition, structural changes can be generated; favoring the exposure of determinants less exposed in the structure, and that can induce higher levels of immune response, as there is a low tolerance of the immune system against these epitopes.

**Example 5. Evaluation of the blocking activity of sera from rats immunized with PKPD-L1 on the binding of PD-1/PD-L1.**

The ability of sera from rats immunized with PKPD-L1 to block the interaction of PD-L1 with its PD-1 receptor was evaluated in a competition ELISA assay. The sera were obtained as has been described in Example 4, at the 1:100 dilution, and incubated for 2 hours at 37 ° C with 500 ng/mL of the PD-L1/Fc protein. This mixture was added to an ELISA plate with the captured PD-1/Fc protein (using a similar condition as the PD-L1 protein was captured in Example 4). The amount of PD-L1 bound to the captured PD-1 molecule was detected with a specific anti PD-L1 biotinylated antibody, and the subsequent addition of a streptavidin-peroxidase conjugate. The maximum binding absorbance value was obtained from the condition in which the interactions of PD-1 with PD-L1 proceed in the absence of any competitor. The inhibition of the interaction between PD-1 with PD-L1, caused by the sera, was expressed as percentage, using the following formula:

$$\% \text{ inhibition} = 100\% - [(\text{A450nm of serum sample} / \text{A450nm maximum binding}) \times 100].$$

Figure 2 shows the results, expressed as percentage, of the serum blocking activity induced in rats immunized with PKPD-L1, PD-L1$^{CHO}$ and the mixture of the PD-L1 peptides on the interaction between the PD-1 receptor and its PD-L1 ligand. The administration of PKPD-L1 in combination with sNAcGM3-VSSP or aluminum phosphate generated an antibody response that had a high capacity to block the binding of the PD-1 receptor with its PD-L1 ligand. This blocking activity was higher, in terms of percentage of inhibition, than those obtained with the antibodies generated by immunization with the PD-L1$^{CHO}$ variant or with the mixture of PD-L1 conjugated peptides (Tukey's test, *p<0.0001*). This result was obtained with the previously mentioned adjuvants.

**Example 6. Evaluation of the ability of sera from rats immunized with PKPD-L1 of blocking the interaction of PD-L1 with monoclonal antibodies specific to said ligand.**

To evaluate whether the antibodies induced after immunization with the chimeric PKPD-L1 antigen are directed to different antigenic determinants of human PD-L1, a competition ELISA was performed. The ability of sera (from the experiment in Example 4) to compete with the monoclonal antibodies atezolizumab (MedChemExpress™), durvalumab (Selleckchem™) and avelumab (MedChemExpress ™) by the binding site on human PD-L1 was evaluated. These anti-PD-L1 monoclonal antibodies (mAbs) are approved by the FDA for the treatment of different tumors, and recognized different epitopes on the PD-L1 molecule (Tan, et al., Protein & cell, 2018: 9: 135-9). To develop the competition ELISA, the monoclonal antibodies were conjugated to biotin, and the EC$_{50}$ of the interaction with PD-L1 and the previously mentioned biotinylated mAbs was determined. The chimeric PD-L1/Fc protein was immobilized on the ELISA plate. Subsequently, the biotinylated mAbs were added in different concentrations, and the antigen-antibody binding was detected with a streptavidin-peroxidase conjugate. For the interactions of PD-L1 with the biotinylated antibodies atezolizumab, durvalumab and avelumab, EC50 of 5.2 ng/mL, 5.8 ng/mL and 3.1 ng/mL, respectively were obtained. Once the conditions were established, the competition ELISA was developed, and immune sera (sera dilution 1:25) compete with the mAbs (at the previously mentioned EC$_{50}$) for human PD-L1 binding sites. The absorbance value corresponding to the maximum binding was obtained using the condition in which the interaction of PD-L1 with each of the monoclonal antibodies occurred in the absence of rat sera. The inhibition on the interaction of monoclonal antibodies with PD-L1 and produced by the sera of rats immunized with PKPD-L1, in combination with aluminum phosphate, was expressed as percentage (using the formula described in Example 5). The results are reflected in Table 3. Sera from rats immunized with 40 mM Tris pH 8.0 (excipient) and aluminum phosphate were used as negative control.

**Table 3.** Ability of the sera of rats immunized with PKPD-L1 to block the interaction between PD-L1 and anti-PD-L1 monoclonal antibodies.

| | % Blocking activity (Media ± SD) | | |
| | atezolizumab | durvalumab | avelumab |
|---|---|---|---|
| **Negative control** | 3.49±2.34% | 3.80±2.67% | 1.67±1.30% |

(continued)

| | % Blocking activity (Media ± SD) | | |
| --- | --- | --- | --- |
| | atezolizumab | durvalumab | avelumab |
| PKPD-L1 | 52.26±4.33% | 57.09±4.84% | 36.11±4.56% |

SD: Standard deviation.

Table 3 shows that administration of the PKPD-L1 polypeptide (in combination with the adjuvant aluminum phosphate) induced specific antibodies that block the binding of PD-L1 with the monoclonal antibodies atezolizumab, durvalumab and avelumab. The blocking activity was significantly different from that obtained in the negative control group (unpaired Student's t-test, *p<0.0001).* After immunization with the PKPD-L1 antigen in combination with an adjuvant, a polyclonal antibody response was induced and is directed to different human PD-L1 epitopes. The immune sera impaired the binding of three anti-PD-L1 monoclonal antibodies that recognize different epitopes. In addition, the immunization induced a polyclonal antibody response that targets relevant epitopes on PD-L1, which are blocked by the monoclonal antibodies atezolizumab, durvalumab and avelumab, approved for the treatment of different tumors.

**Example 7. Evaluation of the capacity to block the PD-1/PD-L1 interaction exhibited by IgG purified from sera of non-human primates immunized with PKPD-L1.**

The aim of the experiment was to evaluate whether the antibodies induced after immunization with the PKPD-L1 polypeptide were able to block the biological effects mediated by the interaction of PD-1 with PD-L1 in a cell-based assay. For this purpose, the Promega ™ J1250 assay was used, which mimics the mechanism of action of biologics that block the interaction between these two molecules. The assay is based on two genetically modified cell lines. The first is the so-called effector cell, which expresses on its cell membrane human PD-1 and a T-cell receptor (TCR) that transduces signals inducing luminescence. The second, the so called Antigen-presenting cells, expresses human PD-L1 and a TCR activator protein. When the two cell types are co-cultured, the PD-1/PD-L1 interaction inhibits the induction of luminescence mediated by TCR signaling. However, the presence of biological inhibitors of the PD-1/PD-L1 axis induces the opposite effect.

To test the ability of the PKPD-L1 polypeptide to induce an immune response in an autologous non-human primate model closely related to human biology, the immunogenicity was evaluated in *Chlorocebus aethiops sabaeus* monkeys. Primates were immunized, subcutaneously with 200 μg of chimeric polypeptide adjuvated with aluminum phosphate. Furthermore, in this experiment was also evaluated the mixture of two PD-L1 synthetic peptides (identified as SEQ ID NO: 23 and SEQ ID NO: 24 in the Sequence List) conjugated to the LpdA protein of *N. meningitidis* (200 μg per dose) adjuvated with aluminum phosphate. The adjuvanted mixtures (each antigen dose was combined with 0.7 mg Al3+) were administered every two weeks, for a total of 4 immunizations. The sera were collected one week after the fourth immunization.

The obtained sera from each experimental group (n=5) were pooled, and the IgGs were purified by protein A affinity chromatography (GE Healthcare™). The cell-based assay was performed according to the manufacturer's instructions, and atezolizumab was used as a positive control for blocking the PD-1/PD-L1 interaction. Furthermore, each condition that was evaluated in the PD-1+ effector cell/PD-L1+ host cell system was also submitted to the PD-1+ effector cell/PD-L1+ host cell negative and maximum luminescence control system. In each case, 6 replicates were evaluated for each condition. Thirty minutes after the simultaneous cultivation of both cell lines, the luminescence was read. In this assay, the blocking activity of the PD-1/PD-L1 interaction is evidenced by the increase in luciferase activity. Consequently, the blocking activity is directly proportional to the increase in luminescence values.

Tabla 4. *In vitro* evaluation of the biological activity of the IgG specific to PD-L1 fraction from non-human primates' serum.

| | Concentration (μg/mL) | Luminescence (RLU) Media ± SD |
| --- | --- | --- |
| IgG Excipient/FA | 500 | 375130 ± 1087 |
| IgG PKPD-L1/FA | 500 | 1335475 ± 46172 |
| IgG MP/FA | 500 | 525030 ± 1401 |
| atezolizumab | 0,1 | 240708 ± 1253 |
| atezolizumab | 20 | 1470587 ± 36617 |

FA: Aluminum phosphate plus excipient Tris 40mM pH 8.0 MP: Mix of conjugated PD-L1 peptides RLU: relative luminescence units.

Table 4 shows that the administration of the high molecular weight aggregates of the PKPD-L1 polypeptide in combination with aluminum phosphate adjuvant, is able to generate biologically active IgG antibodies against human PD-L1, as compared to the negative control group (unpaired Student's t-test, *p<0.0001)*. The luminescence detected for the purified serum IgG fraction and obtained from primates immunized with the PD-L1 polypeptide was 3.56 times higher than the values observed for the negative control group. For the active immunization strategy with PD-L1 peptides, this ratio was 1.4 times higher than the corresponding negative control group (Mann-Whitney test, *p<0.0022)*. These results indicate a superior biological activity of the specific antibodies induced by PKPD-L1 variant as compared to the antibodies generated by PD-L1 peptide mixture. The biological activity of the IgG fraction purified from the sera of monkeys immunized with the PKPD-L1 polypeptide was similar to the atezolizumab positive control at 20 $\mu$g/mL.

**Example 8. Evaluation of the ability of lymphocytes from mice immunized with the PKPD-L1 polypeptide to directly lyse PD-L1-expressing tumor cells.**

In order to evaluate if the PKPD-L1 polypeptide was able to induce a specific cytotoxic T lymphocytes, an assay was developed in which lymphocytes isolated from the spleen of immunocompetent mice were co-cultured with tumor cells. The cytotoxic activity of the lymphocytes was expressed as lysis percentage of the tumor cells. The tumor lines MC38 and CT26, syngeneic with the mouse strains C57Bl6 and Balb /c respectively, were used for the experiment. These cells express PD-L1 on the membrane, and can display peptides of this protein associated with the major type I histocompatibility complex.

The C57Bl6 and Balb/c mice were immunized subcutaneously, with 100 $\mu$g of different PD-L1 variants in combination with the adjuvant sNAcGM3-VSSP (100 $\mu$g). The animals (n=5) received a weekly administration of the PD-L1$^{CHO}$, PKPD-L1 or the mixture of conjugated peptides in combination with the mentioned adjuvant during two months. One week after the last immunization, the spleens were surgically removed, perfused, and the erythrocytes were lysed. Subsequently, the lymphocytes were suspended in RPMI medium supplemented with 10% fetal bovine serum. This lymphocytes (effector cells) were labeled with an anti-CD3 (eBioscience™) antibody and were counted by flow cytometry. The cell lines MC38 and CT26 were labeled with the fluorescent reagent carboxyfluorescein succinimidyl ester (CFSE), resuspended in RPMI medium and used as target cells. CFSE-labeled cells were counted by flow cytometry.

Lymphocytes from C57Bl6 mice ($2\times10^6$ CD3+) were immediately mixed with $2\times10^4$ MC38 cells (100:1 ratio). The lymphocytes isolated from the spleen of Balb/c mice, were first activated *in vitro* for 6 days with 30 $\mu$g/mL of the native PD-L1 protein (PD-L1/Fc), with the addition of 10 units/mL of IL-2 at the third day. After activation, and using the same ratio, effector cells then were co-incubated with CT26 target cells. In both cases, a negative lysis control was used, in which the target cells were incubated with RPMI medium supplemented with fetal bovine serum, instead of adding effector cells. After 4 hours of incubation of the effector cells with the target cells, the mixture was resuspended in PBS and the CFSE-positive target cells were counted by flow cytometry (PARTEC cytometer, Germany). The results are shown in Table 5. Cytotoxicity was expressed as a percentage of lysis of the target cells (tumor cells) according to the expression:

$$\textbf{\% lysis} = 100\% - [(\text{number of CFSE positive target cells incubated with effector cells}) / (\text{number of CFSE positive target cells incubated with medium}) \times 100]$$

**Table 5.** Cytotoxic activity on tumor cells of lymphocytes isolated from the spleen of mice immunized with different variants of PD-L1.

| Immunogens | Percentage of lysis of target cells | |
|---|---|---|
| | MC38 Media $\pm$ SD | CT26 Media $\pm$ SD |
| Excipient + VSSP | 4.72 $\pm$ 4.61% | 1.56 $\pm$ 2.28% |
| PD-L1$^{CHO}$ + VSSP | 21.28 $\pm$ 7.30% | 9.68 $\pm$ 2.60% |
| PKPD-L1 + VSSP | 53.92 $\pm$ 12.09% | 19.82 $\pm$ 4.84% |
| Peptide mixture+ VSSP | 16.62 $\pm$ 3.92% | 3.52 $\pm$ 2.88% |

Excipient: Tris 40 mM pH 8.0. VSSP: sNAcGM3-VSSP. SD: Standard deviation

Table 5 shows that the administration of the PD-L1 polypeptide associated in form of high molecular weight aggregates has a positive effect on cellular immunity. In the experiment with the C57Bl6 strain, a significant increase in the lysis of MC38 tumor cells was detected after the co-incubation with lymphocytes isolated from the spleen of the groups immunized with PD-L1$^{CHO}$ (Tukey's test, *p=0.0169*); with PKPD-L1 (Tukey's test, *p<0.0001*) and with the peptide mixture (Tukey's test, *p=0.0332*) as compared to the control group. The PKPD-L1 polypeptide was able to generate lymphocytes with a cytotoxic activity significantly higher than lymphocytes obtained for the native variant PD-L1$^{CHO}$ (Tukey's test, *p<0.0001*) or for the PD-L1 peptide mixture (Tukey's test, *p<0.0001*).

In the experiment with the Balb/c strain, a significant increase in the lysis of CT26 tumor cells was detected with lymphocytes isolated from the spleen of the groups immunized with PD-L1$^{CHO}$ (Tukey's test, *p=0.0065*) and with PKPD-L1 (Tukey's test, *p<0.0001*) as compared to the control group. Nevertheless, the lymphocytes isolated from the mice immunized with the peptide mixture did not induce cytotoxic activity (Tukey's test, *p=0.7857*). The PKPD-L1 polypeptide also showed superiority in immunogenicity in this mouse strain with different genetic background. After immunization with PKPD-L1, the isolated lymphocytes had higher activity than lymphocytes obtained from mice immunized with the native variant PD-L1$^{CHO}$ (Tukey's test, *p=0.0009*).

**Example 9. Anti-tumor and anti-metastatic effect in the F3II breast carcinoma murine model after the immunization with PD-L1 variants**

The anti-tumor and anti-metastatic activity after the administration of different PD-L1 variants in combination with the adjuvant sNAcGM3-VSSP was evaluated using the F3II breast carcinoma murine model. Female Balb/c mice (9 per group) were immunized subcutaneously with 100 $\mu$g of antigen adjuvanted with sNAcGM3-VSSP as described in Example 8. The F3II cell line was generated from clonal subpopulation of the BALB/c transplantable breast carcinoma with the capacity to spontaneously metastasize to the lung. Two hundred thousand of F3II cells were administered subcutaneously in the dorsal region four days after the fourth immunization at the flank opposite to the immunization site. Once the tumor cells were implanted and after detecting measurable growth, the tumor volume was recorded.

Thirty-five days after the tumor challenge, the mice were euthanized and the lungs were subsequently removed. To count the macrometastases, the lungs were immersed, for at least 48 hours, in Bouin solution (Sigma™). Macrometastases were counted using a stereoscope (Zeiss™). The results are shown in Table 6.

**Table 6.** Tumor volume and number of macrometastases in mice immunized with the PD-L1 variants and challenged with the F3II tumor cell line.

| Treatments | TV (mm$^3$) | Number of metastases |
|---|---|---|
| | Media ± SD | Media ± SD |
| **Excipient + VSSP** | 362 ± 127 | 11.0 ± 4.0 |
| **PD-L1$^{CHO}$ + VSSP** | 222 ± 68 | 2.22 ± 1.92 |
| **PKPD-L1 + VSSP** | 83 ± 18 | 0.67 ± 1.84 |
| **PM + VSSP** | 279 ± 73 | 8.78 ± 4.30 |

Excipient: 40 mM Tris pH 8.0. VSSP: sNAcGM3-VSSP. PM: human PD-L1 peptide mix. TV: Tumor volume. SD: Standard deviation

Table 6 shows the results from tumor volume evaluation eighteen days after challenge with the F3II mouse tumor cells. Administration of the PKPD-L1 polypeptide with the adjuvant sNAcGM3-VSSP significantly reduced tumor growth (Tukey's test, *p<0.0001*), when compared to the negative control group.

Administration of the PD-L1$^{CHO}$ variant also induced a reduction in tumor growth, but with less impact on this parameter (Tukey's test, *p=0.0052*). On the contrary, no decrease in tumor growth was evidenced with the mixture of conjugated peptides (Tukey's test, *p=0.1598*). Immunization with the PKPD-L1 polypeptide caused a greater decrease in tumor volume, as compared to the effect generated by active immunotherapy with PD-L1$^{CHO}$ (Tukey's test, *p<0.0058*).

Table 6 also illustrates the number of spontaneous lung metastases in animals immunized with the different PD-L1 variants. The administration of the fusion polypeptide PKPD-L1 or the variant PD-L1$^{CHO}$ in the context of the adjuvant sNAcGM3-VSSP, inhibited the implantation of metastases in the lung (Tukey test, *p<0.0001*) as compared to the metastases found in the lungs of the animals from control group treated with the adjuvant and the excipient. On the contrary, an effect on the reduction of the implantation of metastases was not evidenced when immunized with the mixture of conjugated peptides (Tukey's test, *p=0.4311*). As for tumor volume, the immune response generated by the PKPD-L1 polypeptide had a superior capacity in reducing the number of metastases, when compared to the effects of the immune response generated after active immunotherapy with the native PD-L1 variant (Tukey's test, *p=0.0004*) or with the mixture of two peptides from this ligand (Tukey's test, *p<0.0001*).

**Example 10. Efficacy of the administration of the PKPD-L1 polypeptide in the treatment of lung metastases in mice.**

Lung metastases are the leading cause of cancer death. Immunization with the PKPD-L1 polypeptide induces a potent humoral and cellular immune responses. The anti-metastatic capacity of the immune response effectors generated after the administration of this fusion polypeptide was evaluated in several models. Groups of 12 mice were immunized with 100 $\mu$g of the polypeptide in combination with 100 $\mu$g of sNAcGM3-VSSP. Mice were immunized during two months, with one dose per week, administered subcutaneously. Tumor challenge was carried out according to the requirements of each model. The analysis of the metastatic load was evaluated through the weight lungs or by metastasis count under a dissecting microscope. For the study in the mouse strain C57Bl/6, $2.5 \times 10^5$ cells of the metastatic lung carcinoma 3LLD122 were inoculated in the footpad, 3 days after the fourth immunization. The primary tumor was removed by surgery 20 days after implantation, and the animals were sacrificed 15 days after surgery, the time expected for the development of lung spontaneous metastases. In this case, the weight of the lungs was determined in each group.

In the second experiment, an experimental metastasis model was used. C57Bl/6 mice were inoculated with murine MB16F10 melanoma cells ($5 \times 10^4$) by the tail vein and four days after the sixth immunization. Seventeen days later, the animals were sacrificed, and black pigmented colonies in the lungs were counted.

In the BALB/c mouse strain, lung metastases were evaluated after intravenous inoculation of $5 \times 10^4$ CT26 colorectal carcinoma cells through the retro-orbital plexus, and administered five days after the fourth immunization. Mice were sacrificed 30 days after tumor challenge and colonies in lungs were counted. The results of these experiments are summarized in Table 7.

**Table 7.** Effect on lung metastasis of the immune response generated after immunization with PKPD-L1 and an adjuvant.

| Tumor Model | Treatment | Lung weight (g) Mean ± SD | Number of macrometastases Mean ± SD |
|---|---|---|---|
| 3LLD122 | Excipients/VSSP | 0.2898±0.06256 | nd |
|  | PKPD-L1/ VSSP | 0.1632±0.03861 | nd |
| MB16F10 | Excipients/VSSP | nd | 61±24 |
|  | PKPD-L1/ VSSP | nd | 42±23 |
| CT26 | Excipients/VSSP | nd | 30 ± 14 |
|  | PKPD-L1/VSSP | nd | 15 ± 9 |

SD: Standard Deviation. VSSP: sNAcGM3-VSSP. nd: Not determined

Comparisons of the results from each PKPD-L1 treated group with respect to its control were carried out independently for each experimental model. As observed in Table 7, in all the studied models, the metastatic load in the lungs from mice immunized with PD-L1 was significantly lower as compared to their control group (Student's t test: 3LLD122, *p<0.0001*; MB16F10, *p=0.0475*; CT26 *p=0.0046*).

**Example 11. Combination of passive and active immunotherapy specific to PD-L1.**

The anti-tumor activity of the combination of passive and active immunotherapy specific to PD-L1 was evaluated in the CT26 murine tumor model. Female Balb/c mice (n= 9 per group) were immunized subcutaneously with 200 $\mu$g of PKPD-L1 polypeptide adjuvanted with 0.7 mg of aluminum phosphate, using the scheme described in the Example 4. Twelve days after the second immunization, $2 \times 10^4$ CT26 cells were inoculated subcutaneously in the dorsal region opposite to the immunization site. One day after the tumor challenge, a PD-L1-blocking antibody (rat IgG2b isotype; clone 10F.9G2; BIOXCELL) was administered once a week to complete a four doses treatment period. The antibody was administered by the intraperitoneal route at the dose of 12,5 mg/kg. For each type of immunotherapy, a negative control group was used. For passive immunotherapy, a control antibody, rat IgG2b isotype (BioXCell™, BE0090) was administered, while for active immunotherapy a group that received PBS and aluminum phosphate adjuvant was used as negative control. After the tumor cells were implanted, and a measurable tumor was detected, the tumor volume was recorded.

Table 8 shown tumor volume measurements, 30 days after challenge with CT26 mouse tumor cells, reflected as mean ± standard deviation.

**Table 8.** Anti-tumor effect of the combination of passive and active immunotherapy targeting PD-L1 in the CT26 colon carcinoma model.

| Treatment | Tumor volume (mm³) |
|---|---|
| Isotype control antibody | 2764 ± 789 |
| Mouse anti-PD-L1 | 1178 ± 988 |
| Mouse anti-PD-L1 /PKPD-L1 + FA | 247 ± 134 |
| PBS + FA | 2717 ± 832 |
| PKPD-L1 + FA | 1046 ± 593 |
| FA: aluminum phosphate. | |

The administration of the rat anti-mouse PD-L1 antibody showed a significant reduction in tumor volume, as compare to the group treated with the isotype control (Dunnett's test, *p=0.003*). Immunization with PKPD-L1 polypeptide also showed a significant effect in reducing tumor volume with respect to the group treated with excipient plus adjuvant (Dunnett's test, *p=0.0001*). The combined treatment showed superior results on tumor shrinkage, compared to PD-L1-based active immunotherapy (Dunnett's test, *p=0.0233*) and passive immunotherapy with anti-PD-L1 antibody (Dunnet's test, *p=0.0332*). These results suggest the possibility of combining passive and active immunotherapies, both targeting PD-L1.

**Example 12. Effect of PKPD-L1 administration on the cell subpopulations in draining lymph nodes and tumor infiltrating lymphocytes.**

To assess whether anti-PD-L1 therapy had an effect on the reversion of PD-L1-induced immunosuppression, the proportions of the cell populations were studied in the lymph nodes and within the tumor. In this experiment, Balb/c mice (n= 9) received two subcutaneous immunizations in 14 days schedule, containing a mixture of 200 $\mu$g of the chimeric PKPD-L1 polypeptide and 0.7 mg of the aluminum phosphate as adjuvant. Three days after the first immunization, mice were challenged subcutaneously with $2 \times 10^4$ colon tumor cells CT26, administered on the opposite side of immunization. Seven days after the second immunization, the mice were sacrificed to extract the tumor and the draining lymph nodes. Membrane PD-1 levels, measured as mean fluorescence intensity, in CD4+ and CD8+ T lymphocytes were evaluated in the sample.

One gram of tumor mass was submitted to enzymatic (eBioscience™) and mechanical dissociation. Dead cells were removed from the tumor suspension with the reagent kit (eBioscience™). The alive cell suspension was subjected to positive leukocyte selection with the selection marker CD45 and the use of magnetic beads (eBioscience™). After elution of the CD45+ cell suspension, lymphocytes were submitted to a multiple immunostaining with anti-CD3, anti-CD4 or anti-CD8 and anti-PD-1 antibodies (eBioscience™). The draining lymph nodes were macerated in RPMI culture medium and the cell suspension was passed through a 30 $\mu$M filter and was subjected to multiple labeling as described for the previous sample. Evaluation of the presence of PD-1 on the membrane of CD4$^+$ and CD8$^+$ T-cell subsets located at draining lymph nodes and within tumor was performed by flow cytometry.

**Table 9.** Analysis of the expression of PD-1 on CD4+ and CD8+ T-cell from lymph nodes and located within tumor.

| Sample | Staining | Fluorescence intensity Mean ± SD | |
|---|---|---|---|
| | | Exc + FA | PKPD-L1 + FA |
| **Draining lymph nodes** | CD3+, CD4+, PD-1+ [high] | 7.946 ± 0.628 | 6.662 ± 1.064 |
| | CD3+, CD8+, PD-1+ [high] | 10.140 ± 1.546 | 7.513 ± 1.436 |
| **Tumor** | CD45+, CD3+, CD4+, PD-1+ | 4.024 ± 0.281 | 3.586 ± 0.348 |
| | CD45+, CD3+, CD8+, PD-1+ | 4.555 ± 0.658 | 3.780 ± 0.559 |
| Exc: Excipient (Tris 40 mM pH 8.0). FA: aluminum phosphate. | | | |

As shown in Table 9; the CD4+ T lymphocytes population within the draining lymph nodes from mice immunized with the adjuvanted PKPD-L1 polypeptide had significantly lower PD-1 membrane levels than those found for the lymphocytes of the mice from the negative control group (unpaired Student's t-test, *p=0.0056*). Similar results were obtained from the analysis of the CD8+ T lymphocytes population (unpaired Student's t-test, *p=0.0014*). This experimental evidence was also obtained for CD4+ (unpaired Student's t-test, *p=0.0156*) and CD8+ (unpaired Student's t-test, *p=0.0240*) tumor infiltrating lymphocytes. Therefore, PD-L1-specific active immunotherapy which the chimeric polypeptide PKPD-L1 as vaccine antigen generates CD4+ and CD8+ T cells with a distinctive phenotype of activated lymphocytes, specifically in relevant immunological sites such as the tumor and draining lymph nodes.

**Example 13. Evaluation of the safety profile after the administration of the PKPD-L1 polypeptide and adjuvant aluminum phosphate or sNAcGM3-VSSP.**

Female Balb/c mice (n= 5) and female New Zealand rabbits (n= 4) were immunized subcutaneously, with 200 μg of the PKPD-L1 chimeric polypeptide. The immunization scheme with VSSP as adjuvant comprised eight weekly vaccinations. In mice, the chimeric polypeptide was adjuvanted with 100 μg of sNAcGM3-VSSP, while for rabbits it was adjuvanted with 200 μg of sNAcGM3-VSSP.

In the case of the combination of PKPD-L1 with aluminum phosphate, 200 μg of the chimeric polypeptide was adjuvanted with 0.7 mg of aluminum phosphate and the doses were administered every 14 days, for a total of four immunizations. For each combination of antigen and adjuvant, the negative control group was immunized with the excipients of each antigen formulation and their respective adjuvant.

In mice experiments, two additional groups of passive immunotherapy were included. One group received four weekly intraperitoneal administrations of a rat antibody specific to mouse PD-L1 (BioXCell™, clone 10F.9G2, 12.5 mg/Kg). The other group was treated with an unrelated antibody of the same isotype (BioXCell™, clone BE0090), using the same dose and schedule.

In both studies, weekly body weight assessments were performed, at different times during immunizations. Once the immunizations were completed, the biological samples were extracted, which could be carried out in 100% of the animals in both animal species.

For mice, at the end of the immunization scheme, the heart, lung, spleen and other ten organs were processed for histopathology. The organs underwent a qualitative evaluation, both macroscopic and microscopic. For microscopic evaluation and histological diagnosis, the samples were processed with each appropriated methodology. It was considered a treatment-related adverse event when alterations were found in the active or passively immunized groups and were not detected in the corresponding placebo group.

For rabbits, two blood samples were collected one before starting the immunization, and the other a week after the last immunization. Each blood extraction was used to obtain plasma (for hematology determinations) and serum (for clinical biochemistry determinations). Hematology determinations included the count and/or percentage of various cell types, among other parameters. Blood biochemistry determinations included hemoglobin concentration, alanine amino transferase enzyme concentration, aspartate aminotransferase enzyme concentration, creatinine concentration, among others. For each parameter, comparisons were made between the values obtained before and after the immunizations, between the treated group and the corresponding placebo group, and were also compared with the reference physiological range reported for the specie. It was considered as a treatment-related adverse event when alterations were found in the groups immunized with the PKPD-L1 polypeptide and were not observed in the corresponding placebo group.

Gross analysis of the removed organs indicated the presence of hemorrhage in the lungs of two of the five mice that received passive immunotherapy with the rat antibody specific to mouse PD-L1. Of these two mice, one of them also had thymus hemorrhage. In the rest of the experimental groups, the organs did not present macroscopic alterations. On the other hand, the microscopic analysis confirmed the hemorrhagic events observed in the lung and thymus that were detected at the macroscopic level in the two aforementioned mice. The other three mice in this group were characterized by having an infiltrate of inflammatory cells in their lungs at the level of the bronchi. In the five mice treated with the specific antibody to mouse PD-L1, all presented mucositis at the level of the colon. The rest of the organs belonging to this group did not show any type of microscopic alteration. In contrast, no microscopic alterations were observed in the organs of the mice belonging to the different placebo groups or in those immunized with the PKPD-L1 polypeptide combined with both adjuvants.

The results of hematology and clinical biochemistry indicated the absence of significant changes between determinations made before and after immunization of rabbits with the PKPD-L1 polypeptide. There were not significant differences between the immunized group and its corresponding placebo group. In general, all the hematology and biochemistry parameters for the immunized and placebo groups were within the physiological range. In both, mice and rabbits, no significant effects were detected on the body weight of the immunized groups with respect to that found in the corresponding placebo groups, even in the different times what was were evaluated.

These results indicate that immunization with the chimeric PKPD-L1 polypeptide, adjuvanted both in sNAcGM3-VSSP or aluminum phosphate generates a safe immune response. This active immunotherapy strategy offers advantages in relation to the passive immunotherapy strategy with specific monoclonal antibodies, due to the lack of adverse effects.

**Claims**

1. Chimeric antigen comprising the extracellular domain of the human programmed death ligand 1 (PD-L1) which forms multimeric aggregates with a reduced binding capacity to the PD-1 and CD80 receptors as compared to the native form of PD-L1.

2. The chimeric antigen of claim 1 comprising an amino-terminal segment to increase its expression in bacteria and a carboxi-terminal segment to facilitate its purification by affinity chromatography.

3. The chimeric antigen of claim 2 having an amino acid sequence identified as SEQ ID NO: 1 or an amino acid sequence that has an identity of at least 95% with SEQ ID NO: 1.

4. A pharmaceutical composition comprising a) a chimeric antigen comprising the extracellular domain of the human programmed death ligand-1 (PD-L1) which forms a multimer that has a reduced capacity to bind the PD-1 and CD80 receptors as compared to the native form of PD-L1 and b) at least one pharmaceutically acceptable vaccine adjuvant.

5. The pharmaceutical composition of claim 4 wherein the vaccine adjuvant is selected from the group composed by oil adjuvants, mineral salts, proteoliposomes, and proteoliposomes conjugated to gangliosides.

6. The composition of claim 5 wherein the vaccine adjuvant is an aluminum salt.

7. Use of a chimeric antigen that comprises the extracellular domain of the human programmed death ligand 1 (PD-L1) which forms a multimer that has a reduced binding capacity to PD-1 and CD80 receptors with respect to the native form of PD-L1 for the manufacture of a drug for the treatment of cancer or its metastases.

8. The use of the chimeric antigen according to claim 7 wherein the drug for the treatment of cancer is a vaccine for active immunotherapy.

9. Method for the treatment of cancer or its metastases in an individual in need, **characterized by** the administration of a therapeutically effective amount of a pharmaceutical composition comprising: a) a chimeric antigen that comprises the extracellular domain of the human programmed death ligand 1 (PD-L1) which forms a multimer that has a reduced binding capacity to PD-1 and CD80 receptors with respect to the native form of PD-L1 and b) at least one pharmaceutically acceptable vaccine adjuvant.

10. The method of claim 9 wherein the administration of the composition comprising the chimeric antigen is simultaneously or sequentially combined with passive immunotherapy or with standard cancer therapy.

11. The method of claim 10 wherein the passive immunotherapy is performed with antibodies against human PD-L1 or against the human programmed cell death protein receptor 1 (PD-1).

# Figure 1

# Figure 2

## INTERNATIONAL SEARCH REPORT

| International application No |
|---|
| PCT/CU2021/050008 |

**A. CLASSIFICATION OF SUBJECT MATTER**
INV. A61P35/00    C07K14/705    C07K16/28
ADD.

According to International Patent Classification (IPC) or to both national classification and IPC

**B. FIELDS SEARCHED**

Minimum documentation searched (classification system followed by classification symbols)
A61P   C07K

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| A | WO 2017/201131 A1 (ALBERT EINSTEIN COLLEGE MEDICINE INC [US]) 23 November 2017 (2017-11-23) Whole document, especially paragraph 78 and the claims | 1-11 |
| A | TERAWAKI SELGO ET AL: "Specific and high-affinity binding of tetramerized PD-L1 extracellular domain to PD-1-expressing cells: possible application to enhance T cell function", INTERNATIONAL IMMUNOLOGY, OXFORD UNIVERSITY PRESS, GB, vol. 19, no. 7, 1 July 2007 (2007-07-01), pages 881-890, XP009126365, ISSN: 0953-8178 Whole document, especially abstract en section methods PD-1 and PD-L1 tetramers. | 1-11 |

-/--

| [X] Further documents are listed in the continuation of Box C. | [X] See patent family annex. |
|---|---|

\* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 14 January 2022 | 28/01/2022 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Kools, Patrick |
|---|---|

Form PCT/ISA/210 (second sheet) (April 2005)

## INTERNATIONAL SEARCH REPORT

| International application No |
| --- |
| PCT/CU2021/050008 |

**C(Continuation).   DOCUMENTS CONSIDERED TO BE RELEVANT**

| Category* | Citation of document, with indication, where appropriate, of the relevant passages | Relevant to claim No. |
| --- | --- | --- |
| A | JINMING YU ET AL:  "PD-L1 expression in human cancers and its association with clinical outcomes", ONCOTARGETS AND THERAPY, vol. Volume 9, 1 August 2016 (2016-08-01), pages 5023-5039, XP055486852, DOI: 10.2147/OTT.S105862 cited in the application the whole document ----- | 1-11 |
| A | MAHONEY KATHLEEN M ET AL:  "A secreted PD-L1 splice variant that covalently dimerizes and mediates immunosuppression", CANCER IMMUNOLOGY IMMUNOTHERAPY, SPRINGER, BERLIN/HEIDELBERG, vol. 68, no. 3, 18 December 2018 (2018-12-18), pages 421-432, XP036736635, ISSN: 0340-7004, DOI: 10.1007/S00262-018-2282-1 [retrieved on 2018-12-18] the whole document ----- | 1-11 |

Form PCT/ISA/210 (continuation of second sheet) (April 2005)

**INTERNATIONAL SEARCH REPORT**

International application No.

PCT/CU2021/050008

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. [X] forming part of the international application as filed:

        [X] in the form of an Annex C/ST.25 text file.

        [ ] on paper or in the form of an image file.

    b. [ ] furnished together with the international application under PCT Rule 13ter.1(a) for the purposes of international search only in the form of an Annex C/ST.25 text file.

    c. [ ] furnished subsequent to the international filing date for the purposes of international search only:

        [ ] in the form of an Annex C/ST.25 text file (Rule 13ter.1(a)).

        [ ] on paper or in the form of an image file (Rule 13ter.1(b) and Administrative Instructions, Section 713).

2. [ ] In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet (1)) (January 2015)

## INTERNATIONAL SEARCH REPORT
Information on patent family members

| International application No |
| --- |
| PCT/CU2021/050008 |

| Patent document cited in search report | Publication date | Patent family member(s) | Publication date |
| --- | --- | --- | --- |
| WO 2017201131 A1 | 23-11-2017 | AU 2017266905 A1 | 22-11-2018 |
| | | CA 3022331 A1 | 23-11-2017 |
| | | CN 109689096 A | 26-04-2019 |
| | | EP 3458095 A1 | 27-03-2019 |
| | | JP 2019522466 A | 15-08-2019 |
| | | KR 20190044029 A | 29-04-2019 |
| | | US 2019153065 A1 | 23-05-2019 |
| | | WO 2017201131 A1 | 23-11-2017 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018167290 A
- WO 2014183649 A
- EP 0816506 B1
- US 6149921 A
- WO 201986056 A1
- EP 0816506 A

### Non-patent literature cited in the description

- **WANG et al.** *OncoTargets and Therapy,* 2016, vol. 9, 5023-39
- **SALMANINEJAD et al.** *Journal of Cellular Physiology,* 2019, vol. 234, 16824-37
- **LEE et al.** *Molecules,* 2019, vol. 24, 1190
- **WANG ; XU.** *JAMIA Open,* 2018, vol. 2, 173-8
- **MUNIR et al.** *Oncoimmunology,* 2013, vol. 2, e23991
- **JØRGENSEN et al.** *HemaSphere,* 2019, vol. 3, 631-2
- **WIECKOWSKI et al.** *Journal of Clinical Oncology,* 2018, vol. 36, 74
- **LIN et al.** *Molecular Therapy Oncolytics,* 2019, vol. 14, 222-32
- **TAN et al.** *Protein & cell,* 2018, vol. 9, 135-9